# EUROPEAN PATENT SPECIFICATION

(11) **EP 2 054 082 B1**
(45) Date of publication and mention of the grant of the patent: **26.12.2012**
(21) Application number: 07811137.4
(22) Date of filing: 07.08.2007
(51) Int. Cl.: A61K 38/17, A61K 38/18, C07K 14/515, C07K 16/28

(54) **Use of Dll4 antagonists in ischemic injury or vascular insufficiency**
Verwendung von Dll4 Antagonisten zur Behandlung von ischaemischen Verletzungen oder von Gefässverengungen
Utilisation d'antagonistes de Dll4 dans le traitement de l' accident ischémique ou de l'insuffisance vasculaire

(30) Priority: 07.08.2006 US 836003 P
(43) Date of publication of application: 06.05.2009
(73) Proprietor: REGENERON PHARMACEUTICALS, INC., Tarrytown, NY 10591 (US)
(72) Inventor: WIEGAND, Stanley, J., Croton-on-Hudson, NY 10520 (US); PAPADOPOULOS, Nicholas, J., Lagrangeville, NY 12540 (US); LOBOV, Ivan, B., New York, NY 10010 (US)
(74) Representative: Bentham, Andrew
(86) International application number: PCT/US2007/017546
(87) International publication number: WO 2008/019144

(56) References cited:
- WO-A-2007/028110
- WO-A-2007/143689
- US-A1- 2006 030 529
- US-A1- 2006 134 121
- FERRARA N ET AL: "Angiogenesis as a therapeutic target", NATURE, NATURE PUBLISHING GROUP, GB, vol. 438, no. 7070, 15 December 2005 (2005-12-15), pages 967-674, XP008084462, ISSN: 0028-0836, DOI: 10.1038/NATURE04483
- NOGUERA-TROISE IRENE ET AL: "Blockade of Dll4 inhibits tumour growth by promoting non-productive angiogenesis", NATURE, NATURE PUBLISHING GROUP, GB, vol. 444, no. 7122, 21 December 2006 (2006-12-21), pages 1032-1037, XP002489427, ISSN: 0028-0836, DOI: 10.1038/NATURE05355

### SEQUENCE LISTING

<110> Regeneron Pharmaceuticals, Inc
<120> Therapeutic Methods for Treating Vascular Eye Disorders with DLL4 Antagonists
<130> 3090A-WO
<140> To be assigned
   <141> 2007-08-07
<150> 60/836,003
   <151> 2006-08-07
<160> 5
<170> FastSEQ for Windows Version 4.0
<210> 1
   <211> 2058
   <212> DNA
   <213> Homo sapien
<400> 1
<210> 2
   <211> 685
   <212> PRT
   <213> Homo sapien
<400> 2
<210> 3
   <211> 3427
   <212> DNA
   <213> Homo sapien
<400> 3
   ctcgcaggct aggaacccga ggccaagagc tgcagccaaa gtcacttggg tgcagtgtac 60
   tccctcacta gcccgctcga gaccctagga tttgctccag gacacgtact tagagcagcc 120
   accgcccagt cgccctcacc tggattacct accgaggcat cgagcagcgg agtttttgag 180
   aaggcgacaa gggagcagcg tcccgagggg aatcagcttt tcaggaactc ggctggcaga 240
   cgggacttgc gggagagcga catccctaac aagcagattc ggagtcccgg agtggagagg 300
   acaccccaag ggatgacgcc tgcgtcccgg agcgcctgtc gctgggcgct actgctgctg 360
   gcggtactgt ggccgcagca gcgcgctgcg ggctccggca tcttccagct gcggctgcag 420
   gagttcgtca accagcgcgg tatgctggcc aatgggcagt cctgcgaacc gggctgccgg 480
   actttcttcc gcatttgcct taagcacttc caggcaacct tctccgaggg accctgcacc 540
   tttggcaatg tctccacgcc ggtattgggc accaactcct tcgtcgtcag ggacaagaat 600
   agcggcagtg gtcgcaaccc tctgcagttg cccttcaatt tcacctggcc gggaaccttc 660
   tcactcaaca tccaagcttg gcacacaccg ggagacgacc tgcggccaga gacttcgcca 720
   ggaaactctc tcatcagcca aatcatcatc caaggctctc ttgctgtggg taagatttgg 780
   cgaacagacg agcaaaatga caccctcacc agactgagct actcttaccg ggtcatctgc 840
   agtgacaact actatggaga gagctgttct cgcctatgca agaagcgcga tgaccacttc 900
   ggacattatg agtgccagcc agatggcagc ctgtcctgcc tgccgggctg gactgggaag 960
   tactgtgacc agcctatatg tctttctggc tgtcatgagc agaatggtta ctgcagcaag 1020
   ccagatgagt gcatctgccg tccaggttgg cagggtcgcc tgtgcaatga atgtatcccc 1080
   cacaatggct gtcgtcatgg cacctgcagc atcccctggc agtgtgcctg cgatgaggga 1140
   tggggaggtc tgttttgtga ccaagatctc aactactgta ctcaccactc tccgtgcaag 1200
   aatggatcaa cgtgttccaa cagtgggcca aagggttata cctgcacctg tctcccaggc 1260
   tacactggtg agcactgtga gctgggactc agcaagtgtg ccagcaaccc ctgtcgaaat 1320
   ggtggcagct gtaaggacca ggagaatagc taccactgcc tgtgtccccc aggctactat 1380
   ggccagcact gtgagcatag taccttgacc tgtgcggact caccctgctt caatgggggc 1440
   tcttgccggg agcgcaacca ggggtccagt tatgcctgcg aatgcccccc caactttacc 1500
   ggctctaact gtgagaagaa agtagacagg tgtaccagca acccgtgtgc caatggaggc 1560
   cagtgcctga acagaggtcc aagccgaacc tgccgctgcc ggcctggatt cacaggcacc 1620
   cactgtgaac tgcacatcag cgattgtgcc cgaagtccct gtgcccacgg gggcacttgc 1680
   cacgatctgg agaatgggcc tgtgtgcacc tgccccgctg gcttctctgg caggcgctgc 1740
   gaggtgcgga taacccacga tgcctgtgcc tccggaccct gcttcaatgg ggccacctgc 1800
   tacactggcc tctccccaaa caacttcgtc tgcaactgtc cttatggctt tgtgggcagc 1860
   cgctgcgagt ttcccgtggg cttgccaccc agcttcccct gggtagctgt ctcgctgggc 1920
   gtggggctag tggtactgct ggtgctgctg gtcatggtgg tagtggctgt gcggcagctg 1980
   cggcttcgga ggcccgatga cgagagcagg gaagccatga acaatctgtc agacttccag 2040
   aaggacaacc taatccctgc cgcccagctc aaaaacacaa accagaagaa ggagctggaa 2100
   gtggactgtg gtctggacaa gtccaattgt ggcaaactgc agaaccacac attggactac 2160
   aatctagccc cgggactcct aggacggggc agcatgcctg ggaagtatcc tcacagtgac 2220
   aagagcttag gagagaaggt gccacttcgg ttacacagtg agaagccaga gtgtcgaata 2280
   tcagccattt gctctcccag ggactctatg taccaatcag tgtgtttgat atcagaagag 2340
   aggaacgagt gtgtgattgc cacagaggta taaggcagga gcctactcag acacccagct 2400
   ccggcccagc agctgggcct tccttctgca ttgtttacat tgcatcctgt atgggacatc 2460
   tttagtatgc acagtgctgc tctgcggagg aggagggaat ggcatgaact gaacagactg 2520
   tgaacccgcc aagagttgca ccggctctgc acacctccag gagtctgcct ggcttcagat 2580
   gggcagcccc gccaagggaa cagagttgag gagttagagg agcatcagtt gagctgatat 2640
   ctaaggtgcc tctcgaactt ggacttgctc tgccaacagt ggtcatcatg gagctcttga 2700
   ctgttctcca gagagtggca gtggccctag tgggtcttgg cgctgctgta gctcctgtgg 2760
   gcatctgtat ttccaaagtg cctttgccca gactccatcc tcacagctgg gcccaaatga 2820
   gaaagcagag aggaggcttg caaaggatag gcctcccgca ggcagaacag ccttggagtt 2880
   tggcattaag caggagctac tctgcaggtg aggaaagccc gaggagggga cacgtgtgac 2940
   tcctgcctcc aaccccagca ggtggggtgc cacctgcagc ctctaggcaa gagttggtcc 3000
   ttcccctggt cctggtgcct ctgggctcat gtgaacagat gggcttaggg cacgcccctt 3060
   ttgccagcca ggggtacagg cctcactggg gagctcaggg ccttcatgct aaactcccaa 3120
   taagggagat ggggggaagg gggctgtggc ctaggccctt ccctccctca cacccatttt 3180
   tgggcccttg agcctgggct ccaccagtgc ccactgttgc cccgagacca accttgaagc 3240
   cgattttcaa aaatcaataa tatgaggttt tgttttgtag tttattttgg aatctagtat 3300
   tttgataatt taagaatcag aagcactggc ctttctacat tttataacat tattttgtat 3360
   ataatgtgta tttataatat gaaacagatg tgtacataaa aaaaaaaaaa aaaaaaaaaa 3420
   aaaaaaa                                                           3427
<210> 4
   <211> 686
   <212> PRT
   <213> Homo sapien
<400> 4
   Met Thr Pro Ala Ser Arg Ser Ala Cys Arg Trp Ala Leu Leu Leu Leu
   1               5                   10                  15
   Ala Val Leu Trp Pro Gln Gln Arg Ala Ala Gly Ser Gly Ile Phe Gln
               20                  25                  30
   Leu Arg Leu Gln Glu Phe Val Asn Gln Arg Gly Met Leu Ala Asn Gly
           35                  40                  45
   Gln Ser Cys Glu Pro Gly Cys Arg Thr Phe Phe Arg Ile Cys Leu Lys
       50                  55                  60
   His Phe Gln Ala Thr Phe Ser Glu Gly Pro Cys Thr Phe Gly Asn Val
   65                  70                  75                  80
   Ser Thr Pro Val Leu Gly Thr Asn Ser Phe Val Val Arg Asp Lys Asn
                   85                  90                  95
   Ser Gly Ser Gly Arg Asn Pro Leu Gln Leu Pro Phe Asn Phe Thr Trp
               100                 105                 110
   Pro Gly Thr Phe Ser Leu Asn Ile Gln Ala Trp His Thr Pro Gly Asp
           115                 120                 125
   Asp Leu Arg Pro Glu Thr Ser Pro Gly Asn Ser Leu Ile Ser Gln Ile
           130                 135                 140
   Ile Ile Gln Gly Ser Leu Ala Val Gly Lys Ile Trp Arg Thr Asp Glu
   145                 150                 155                 160
   Gln Asn Asp Thr Leu Thr Arg Leu Ser Tyr Ser Tyr Arg Val Ile Cys
                   165                 170                 175
   Ser Asp Asn Tyr Tyr Gly Glu Ser Cys Ser Arg Leu Cys Lys Lys Arg
               180                 185                 190
   Asp Asp His Phe Gly His Tyr Glu Cys Gln Pro Asp Gly Ser Leu Ser
           195                 200                 205
   Cys Leu Pro Gly Trp Thr Gly Lys Tyr Cys Asp Gln Pro Ile Cys Leu
       210                 215                 220
   Ser Gly Cys His Glu Gln Asn Gly Tyr Cys Ser Lys Pro Asp Glu Cys
   225                 230                 235                 240
   Ile Cys Arg Pro Gly Trp Gln Gly Arg Leu Cys Asn Glu Cys Ile Pro
                   245                 250                 255
   His Asn Gly Cys Arg His Gly Thr Cys Ser Ile Pro Trp Gln Cys Ala
               260                 265                 270
   Cys Asp Glu Gly Trp Gly Gly Leu Phe Cys Asp Gln Asp Leu Asn Tyr
           275                 280                 285
   Cys Thr His His Ser Pro Cys Lys Asn Gly Ser Thr Cys Ser Asn Ser
       290                 295                 300
   Gly Pro Lys Gly Tyr Thr Cys Thr Cys Leu Pro Gly Tyr Thr Gly Glu
   305                 310                 315                 320
   His Cys Glu Leu Gly Leu Ser Lys Cys Ala Ser Asn Pro Cys Arg Asn
                   325                 330                 335
   Gly Gly Ser Cys Lys Asp Gln Glu Asn Ser Tyr His Cys Leu Cys Pro
               340                 345                 350
   Pro Gly Tyr Tyr Gly Gln His Cys Glu His Ser Thr Leu Thr Cys Ala
           355                 360                 365
   Asp Ser Pro Cys Phe Asn Gly Gly Ser Cys Arg Glu Arg Asn Gln Gly
       370                 375                 380
   Ser Ser Tyr Ala Cys Glu Cys Pro Pro Asn Phe Thr Gly Ser Asn Cys
   385                 390                 395                 400
   Glu Lys Lys Val Asp Arg Cys Thr Ser Asn Pro Cys Ala Asn Gly Gly
                   405                 410                 415
   Gln Cys Leu Asn Arg Gly Pro Ser Arg Thr Cys Arg Cys Arg Pro Gly
               420                 425                 430
   Phe Thr Gly Thr His Cys Glu Leu His Ile Ser Asp Cys Ala Arg Ser
           435                 440                 445
   Pro Cys Ala His Gly Gly Thr Cys His Asp Leu Glu Asn Gly Pro Val
       450                 455                 460
   Cys Thr Cys Pro Ala Gly Phe Ser Gly Arg Arg Cys Glu Val Arg Ile
   465                 470                 475                 480
   Thr His Asp Ala Cys Ala Ser Gly Pro Cys Phe Asn Gly Ala Thr Cys
                   485                 490                 495
   Tyr Thr Gly Leu Ser Pro Asn Asn Phe Val Cys Asn Cys Pro Tyr Gly
               500                 505                 510
   Phe Val Gly Ser Arg Cys Glu Phe Pro Val Gly Leu Pro Pro Ser Phe
           515                 520                 525
   Pro Trp Val Ala Val Ser Leu Gly Val Gly Leu Val Val Leu Leu Val
       530                 535                 540
   Leu Leu Val Met Val Val Val Ala Val Arg Gln Leu Arg Leu Arg Arg
   545                 550                 555                 560
   Pro Asp Asp Glu Ser Arg Glu Ala Met Asn Asn Leu Ser Asp Phe Gln
                   565                 570                 575
   Lys Asp Asn Leu Ile Pro Ala Ala Gln Leu Lys Asn Thr Asn Gln Lys
               580                 585                 590
   Lys Glu Leu Glu Val Asp Cys Gly Leu Asp Lys Ser Asn Cys Gly Lys
           595                 600                 605
   Leu Gln Asn His Thr Leu Asp Tyr Asn Leu Ala Pro Gly Leu Leu Gly
       610                 615                 620
   Arg Gly Ser Met Pro Gly Lys Tyr Pro His Ser Asp Lys Ser Leu Gly
   625                 630                 635                   640
   Glu Lys Val Pro Leu Arg Leu His Ser Glu Lys Pro Glu Cys Arg Ile
                   645                 650                 655
   Ser Ala Ile Cys Ser Pro Arg Asp Ser Met Tyr Gln Ser Val Cys Leu
               660                 665                 670
   Ile Ser Glu Glu Arg Asn Glu Cys Val Ile Ala Thr Glu Val
           675                 680                 685
<210> 5
   <211> 227
   <212> PRT
   <213> Homo sapiens
<400> 5
   Asp Lys Thr His Thr Cys Pro Pro Cys Pro Ala Pro Glu Leu Leu Gly
    1               5                  10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225

## Description

### Field of the invention

This invention relates generally to treatment of vascular and ischemic disorders by administering compositions that inhibit DII4 interaction with Notch receptors, and/or Notch receptor activation. More specifically, this invention relates to treatment of eye vascular and/or ischemic disorders by administering compositions that inhibit DII4 Interaction with Notch receptors.

### Background

Angiogenesis is a fundamental process required for the normal development and growth of tissues and organs, and involves formation of new blood vessels from pre-existing vessels. Angiogenesis and blood vessel homeostasis are tightly controlled through a highly regulated system of angiogenic modulators. Deviation from such a tight control often leads to or is associated with disease.

While unregulated "excessive" or aberrant angiogenesis is characteristic of numerous disease states, insufficient angiogenesis, loss of blood vessels or functional or structural blood vessel occlusion can also be a serious medical problem. Promoting angiogenesis and/or preventing regression of existing blood vessels is desirable in situations where tissue becomes ischemic, for example in retinal, cerebral, cardiovascular and limb ischemia and in conditions where a vascular supply must be established, re-established, enhanced or expanded, for example in wound healing and after tissue or organ transplantation, or to stimulate establishment of collateral vessels or otherwise increase the perfusion of a tissue or organ with inadequate circulation.

The Notch-signaling pathway is a system for cell-to-cell communication used by a wide range of eukaryotes for many biological processes, such as differentiation, proliferation, and homeostasis (Artavanis-Tsakonas et al. (1999) Science 284:770-776). Notch signaling has also been implicated in the control of vascular development (Iso et al. (2003) Arterioscler Thromb Vasc Biol. 23:543-553).

Delta-like 4 (DI4) or delta-like ligand 4 (DII4) (hereinafter "DII4") is a member of the Delta family of Notch ligands which exhibits highly selective expression by vascular endothelium (Shutter et al. (2000) Genes Dev. 14:1313-1318). DII4 is a ligand for Notch receptors, including Notch1 and Notch 4. The nucleic acid and amino acid sequences for human and mouse DII4 are shown in SEQ ID NO:1-2 and SEQ ID NO:3-4, respectively. Gene-targeted DII4 mice have been generated (see, for example, Duarte et al. (2004) Genes Dev. 18:2474 - 2478; Krebs et al. (2004) Genes Dev. 18:2469 - 2473: Gale et al. (2004) Proc Natl Acad Sci USA 101:15949-15954). These studies showed that DII4 was highly expressed on developing blood vessels in the mouse embryo, and that genetic deletion of even a single DII4 allele resulted in embryonic lethality, associated with marked vascular abnormalities.

US 2006/134121 discloses DII4 antagonists, assays, and therapeutic methods thereof. US 2006/030529 discloses use of VEGF inhibitors for treatment of eye disorders. WO 2007/028110 discloses methods for using and identifying modulators of delta-like 4. WO 2007/143689 discloses compositions and methods for modulating vascular development.

### BRIEF SUMMARY OF THE INVENTION

The present invention relates to treating diseases affecting the cardiovascular system with agents capable of blocking the interaction of DII4 with its receptors ("DII4 antagonists"). The experimental results provided herein support the use of DII4 antagonists for treating diseases characterized by pathological neovascularization and vascular insufficiency, where vascular insufficiency is due to the loss of blood vessels or functional vascular changes resulting in insufficient tissue perfusion. DII4 antagonists are particularly useful for treating vascular disorders of the eye, for example, diabetic retinopathy, retinopathy of prematurity and other ocular diseases characterized by pathological angiogenesis and/or retinal ischemia, such as retinal vein or artery occlusion.

The invention provides use of a delta-like ligand 4 (DII4) antagonist in the manufacture of a medicament for preventing or reducing blood vessel loss or promoting productive angiogenesis, or both, in a subject having an ischemic injury or vascular insufficiency wherein the DII4 antagonist comprises an antibody or antibody fragment that specifically binds DII4 and blocks the binding of DII4 to a Notch receptor or comprises the extracellular domain of DII4, optionally connected to a multimerizing component. The invention also provides a delta-like ligand 4 (DII4) antagonist for use in preventing or reducing blood vessel loss or promoting productive angiogenesis, or both, in a subject having an ischemic injury or vascular insufficiency, wherein the DII4 antagonist comprises an antibody or antibody fragment that specifically binds DII4 and blocks the binding of DII4 to a Notch receptor or comprises the extracellular domain of DII4, optionally connected to a multimerizing component.

Pharmacological inhibition of DII4 is shown to exert several beneficial effects in a model of ischemic retinopathy, including attenuation of blood vessel loss when applied at the time of an injury leading to blood vessel loss, and suppression of the development of pathological changes in the vasculature while enhancing the regrowth of more normal, functional blood vessels when applied following the establishment of ischemia.

Pharmacological agents which inhibit DII4 signaling are expected to be similarly beneficial in treating other forms of ischemic injury, including cerebral ischemia, cardiac ischemia, and ischemic conditions affect the limbs and other body organs or tissues. DII4 inhibitors are also expected to be clinically beneficial in other conditions requiring the establishment or re-establishment of a vascular supply or that would otherwise benefit from enhanced tissue perfusion. Examples of such conditions are arteriovenous malformations, wound healing, organ or tissue transplantation, and placental insufficiency. As shown in the experimental work reported below, DII4 inhibition prevents arterial narrowing and occlusion, indicating that DII4 inhibitors would also be effective in treating systemic or pulmonary hypertension, and related disorders.

DII4 antagonists are now shown to be effective in promoting productive angiogenesis in both normal and pathological conditions. Specifically, blocking DII4-Notch signaling is shown to enhance angiogenic sprouting and vascular endothelial cell proliferation in the developing retinal vasculature, and to suppress formation of pathological, ectopic neovascularization and the development of arteriovenous shunts in favor of more appropriate vessel formation in the ischemic retina. Moreover, the application of DII4 blockers at the time of vessel injury is shown to markedly reduce the subsequent loss of blood vessels. These findings support the use of a DII4 antagonist in the treatment of eye diseases characterized by vascular abnormalities, especially when accompanied by ischemia and loss of normal vessels. Examples of such eye diseases include ischemic retinopathies, such as age-related macular degeneration, central retinal vein occlusion or branch retinal vein occlusion, diabetic retinopathy, and retinopathy of prematurity.

Also described herein is a method of treating an eye disorder or disease characterized by vascular abnormalities, comprising administering a DII4 antagonist to a subject in need thereof. The method promotes growth of functional normal vessels, inhibits the growth of abnormal or disordered vessels and prevents pathological regression of blood vessels.

In one embodiment, the DII4 antagonist is an antibody or antibody fragment which specifically binds DII4 and blocks DII4 binding to a Notch receptor (for example, Notch1 and Notch 4 receptors). In another embodiment, the DII4 antagonist is a protein or protein fragment comprising the extracellular domain of DII4 or a fragment thereof, which in specific embodiments may be fused to a multimerizing component and which binds Notch receptors without activating them, thereby blocking the actions of endogenous DII4.

The eye disorder or disease treated by the method is a disorder of the ocular vasculature characterized by the presence of abnormal vessels and/or loss of normal vessels or normal vessel function. In specific embodiments, the condition or disorder treated includes retinopathy of prematurity, ischemic retinopathy, retinal vein or artery occlusion, diabetic retinopathy, choroidal neovascularization, age related macular degeneration, corneal neovascularization, neovascular glaucoma or corneal transplantation.

The antibody or antibody fragment used in the method may be polyclonal or monoclonal, and may be humanized, chimeric, or fully human. Preferably the antibody is a fully human monoclonal antibody or monoclonal antibody fragment. The antibody fragment may be a single chain antibody, ScFv, an Fab, or an F(ab')₂.

When the DII4 antagonist is a protein or protein fragment, the fragment is preferably the extracellular domain of DII4 or a fragment or modified fragment thereof, and may be fused to a multimerizing component. The multimerizing component is preferably an immunoglobulin domain, such as for example an Fc domain, e.g., a human Fc (SEQ ID NO:5). The protein may optionally comprise a signal sequence, which may be native to the cell, recombinant, or synthetic.

In a broader aspect, the method is useful to treat any ischemic disease or condition caused by insufficient blood supply due to blood vessel loss and/or poor perfusion, for example, ischemic injury, cerebral ischemia, cardiac ischemia, ischemic conditions affecting the limbs and other organs or tissues, arteriovenous malformations, wound healing, organ or tissue transplantation, placental insufficiency, arterial narrowing and occlusion, atherosclerosis, and systemic or pulmonary hypertension.

Also described herein is the use of an agent capable of inhibiting DII4 activity in the manufacture of a medicament for treating, inhibiting or ameliorating an ischemic or vascular disorder, wherein the DII4 antagonist is an antibody or antibody fragment capable of blocking the binding of DII4 to a Notch receptor, or a DII4 fragment optionally connected to a multimerizing component (such as an Fc domain). In one embodiment, the ischemic or vascular disorder is an eye disease or condition characterized by the presence of abnormal blood vessels and/or loss of normal blood vessels or vessel function. The eye disease is retinopathy of prematurity, ischemic retinopathy, retinal vein or artery occlusion, diabetic retinopathy, choroidal neovascularization, age related macular degeneration, corneal neovascularization, neovascular glaucoma or corneal transplantation.

In another embodiment, the ischemic or vascular disorder is ischemic injury, cerebral ischemia, cardiac ischemia, ischemic conditions affecting the limbs and other organs or tissues, arteriovenous malformations, wound healing, organ or tissue transplantation, placental insufficiency, arterial narrowing and occlusion, atherosclerosis, or systemic or pulmonary hypertension.

Other objects and advantages will become apparent from a review of the ensuing detailed description.

### BRIEF SUMMARY OF THE FIGURES

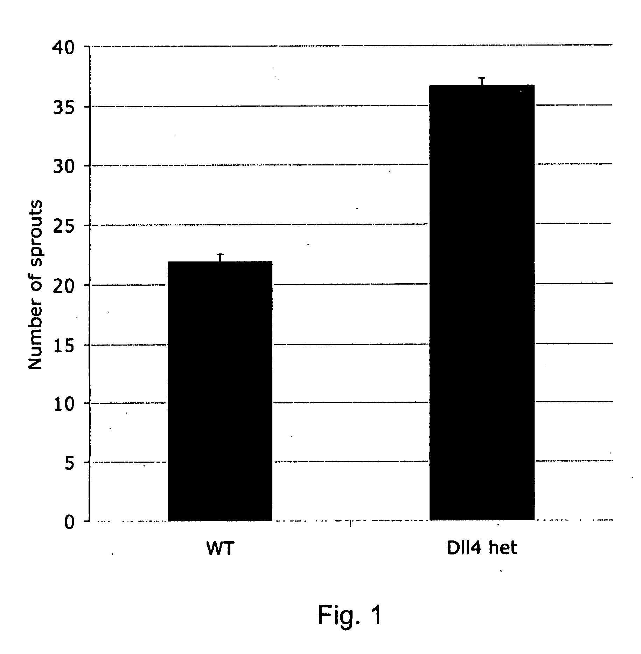

Fig. 1. Genetic deletion of a single DII4 allele increased numbers of angiogenic sprouts in the developing retinal vasculature. Numbers of sprouts were quantified in 100X microscopy fields. Results were significantly different at the p<0.00001 level.

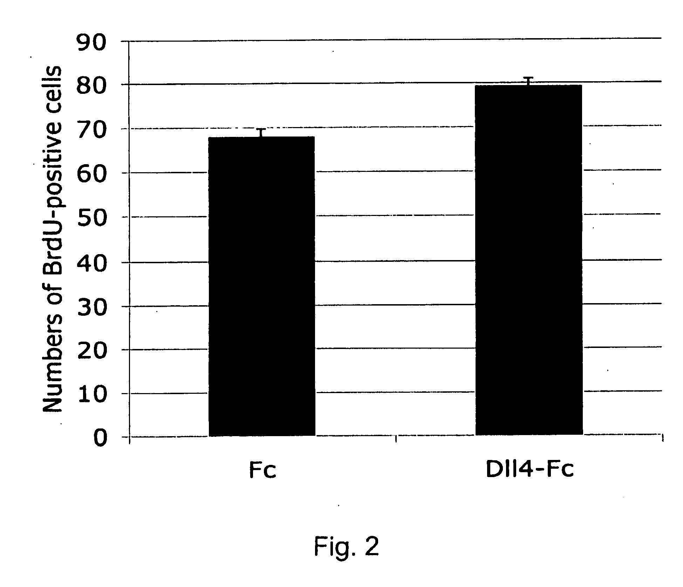

Fig. 2. Intraocular delivery of DII4-Fc increased numbers of proliferating BrdU-positive cells in the developing retinal vasculature. 4.15 mcg of mDII4-hFc or 5 mcg of human hFc control protein was injected intravitreally in 7 days old mouse pups. Retinal vasculature was analyzed 24 hours later. Numbers of BrdU positive cells were quantified in 200X microscopy fields. Results were significantly different at the p<0.05 level.

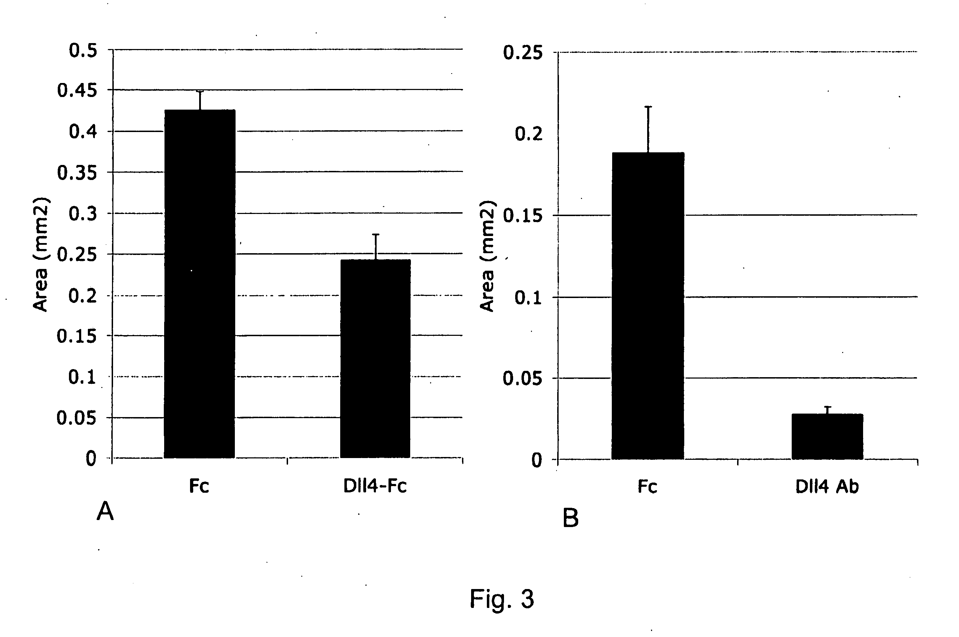

Fig. 3A-B. Intraocular delivery of DII4-Fc or anti-DII4 antibody promotes the regrowth of retinal vessels in mice with oxygen-induced ischemic retinopathy (OIR). (A) 0.48 mcg of mDII4-hFc or 0.5 mcg of human hFc control protein was injected intravitreally in 13 days old (postnatal day 13, or P13) OIR pups. Retinal vasculature was analyzed at P17. (B) 2.55 mcg of rabbit polyclonal anti-mDII4 antibody or 5 mcg of human hFc control protein was injected intravitreally at P13. Retinal vasculature was analyzed at P17. Avascular areas were measured in retinal flat-mounts. Results were significantly different at the p<0.0001 (A) and p<0.05 (B) levels.

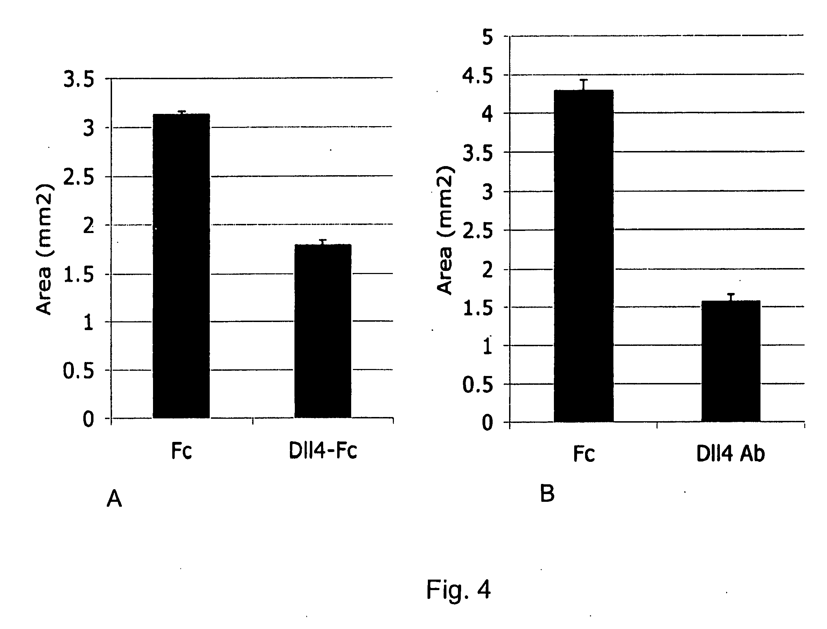

Fig. 4A-B. Intraocular delivery of DII4-Fc or anti-DII4 antibody ameliorates formation of pathological neovascularization in OIR. (A) 0.48 mcg of mDII4-hFc or 0.5 mcg of human hFc control protein was injected intravitreally at P13. Retinal vasculature was analyzed at P17. (B) 2.55 mcg of rabbit polyclonal anti-mDII4 antibody or 5 mcg of human hFc control protein was injected intravitreally at P13. Retinal vasculature was analyzed at P17. Abnormal vascular areas (areas containing ectopic vascular "tufts") were measured in retinal flat-mounts. Results were significantly different at the p<0.0001 (A) and p<0.05 (B) levels.

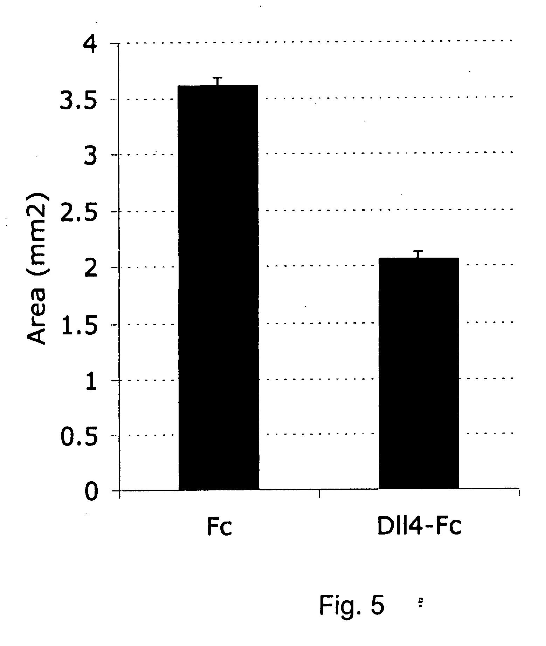

Fig. 5. Intraocular delivery of DII4-Fc improves retinal perfusion. 0.48 mcg of mDII4-hFc or 0.5 mcg of human hFc control protein was injected intravitreally at P13. At P17 animals were perfused with tomato lectin labeled with Texas Red and retinal vasculature was analyzed at P17. Non-perfused vascular areas were measured in retinal flat-mounts. Results were significant at the p<0.0005 level.

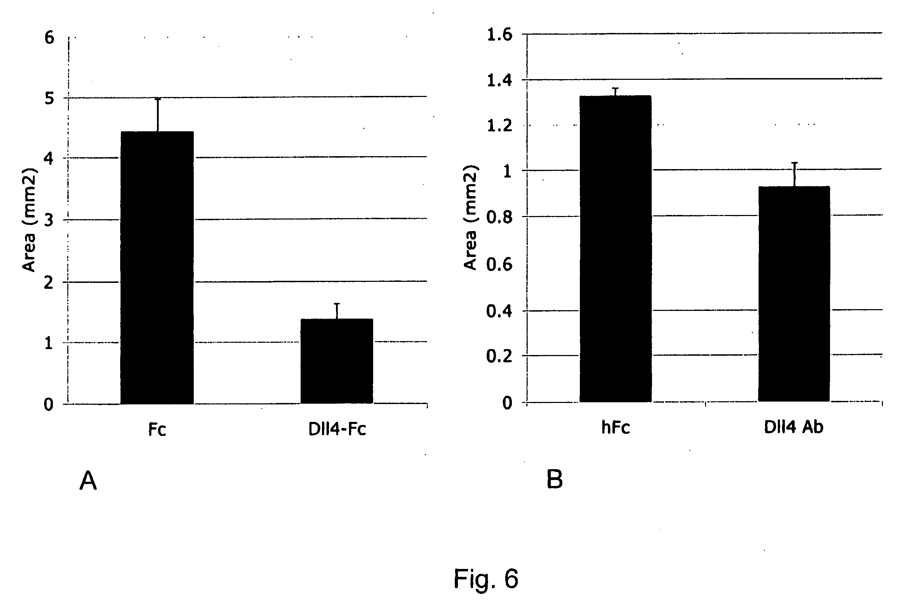

Fig. 6A-B. Intraocular delivery of DII4-Fc or anti-DII4 antibody reduces retinal hypoxia/ischemia. (A) 4.1 mcg of hDII4-hFc or 5 mcg of human hFc control protein was injected intravitreally at P12. Hypoxyprobe™-1 was injected intraperitoneally one hour before sacrificing the animals. Retinal vasculature was analyzed at P17. Hypoxyprobe-labeled areas were measured in retinal flat-mounts. (B) 2.55 mcg of rabbit polyclonal anti-mDII4 antibody or 5 mcg of human hFc control protein was injected intravitreally at P13. Hypoxyprobe™-1 was injected intraperitoneally one hour before sacrificing the animals. Retinal vasculature was analyzed at P17. Hypoxyprobe-labeled areas were measured in retinal flat-mounts. Results were significantly different at the p<0.001 (A) and p<0.05 (B) levels.

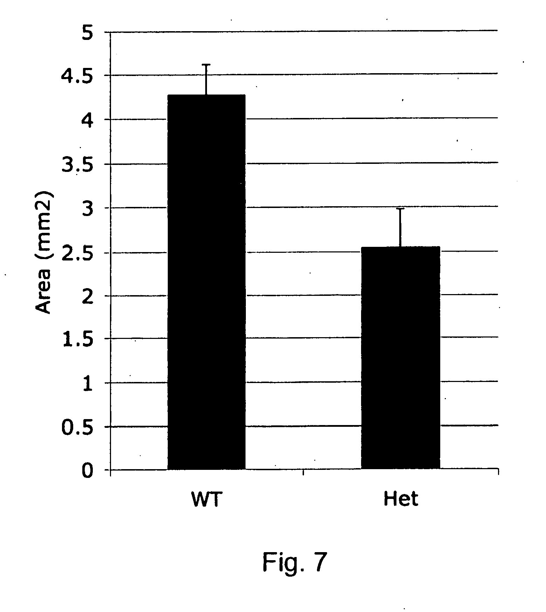

Fig. 7. Genetic deletion of a single DII4 allele reduces blood vessel loss induced by exposure to hyperoxia. DII4^{+/lacZ} and littermate WT control mice were placed into the 75% oxygen chamber at P7. Retinal vasculature was analyzed in flat-mounts at P12. Results were significantly different at the p<0.05 levels.

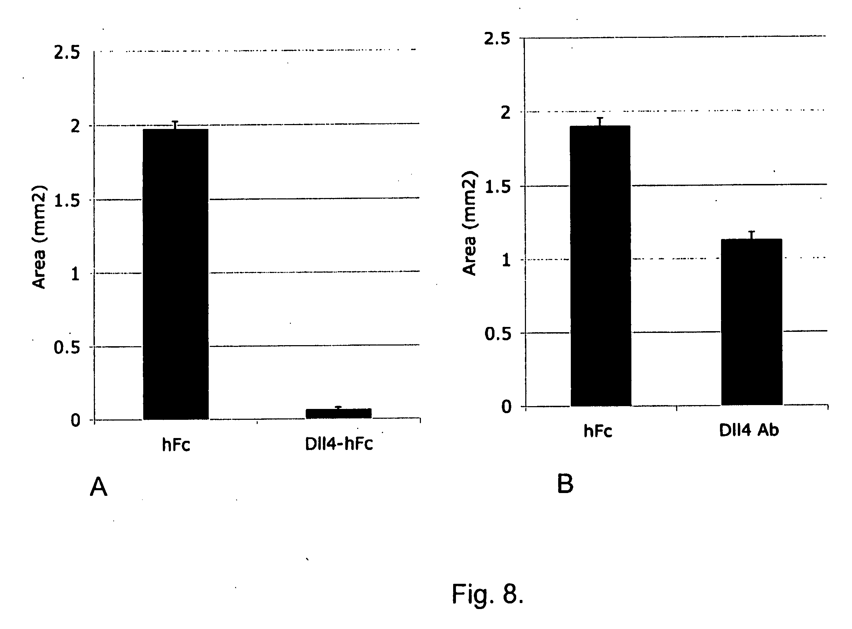

Fig. 8A-B. Intraocular delivery of DII4-Fc or anti-DII4 antibody reduces or prevents blood vessel loss induced by exposure to hyperoxia. (A) 4.1 mcg of hDII4-hFc or 5 mcg of human hFc control protein was injected intravitreally at P8. Pups were placed into a 75% oxygen environment at P9. Retinas vasculature was analyzed at P10. (B) 2.55 mcg of rabbit polyclonal anti-mDII4 antibody or 5 mcg of human hFc control protein was injected intravitreally at P8. Pups were placed into a 75% oxygen environment at P9. Retinal vasculature was analyzed at P10. Results were significantly different at the p<0.00001 (A) and p<0.0001 (B) levels.

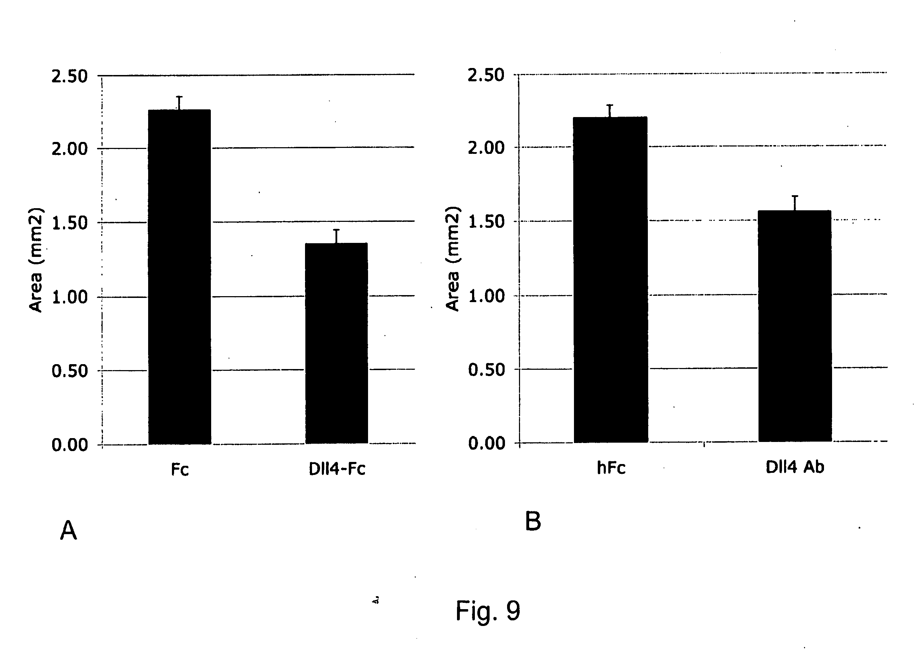

Fig. 9A-B. Intraocular delivery of DII4-Fc or anti-DII4 antibody reduces retinal vessel occlusion. (A) 4.1 mcg of hDII4-hFc or 5 mcg of human hFc control protein was injected intravitreally at P8. Pups were placed into a 75% oxygen environment at P9. Pups were perfused with fluorescent lectin and retinal vasculature was analyzed at P10. (B) 2.55 mcg of rabbit polyclonal anti-mDII4 antibody or 5 mcg of human hFc control protein was injected intravitreally at P8. Pups were placed into a 75% oxygen environment at P9. Pups were perfused with fluorescent lectin and retinal vasculature was analyzed at P10. Results were significantly different at the p<0.00001 (A) and p<0.0001 (B) levels.

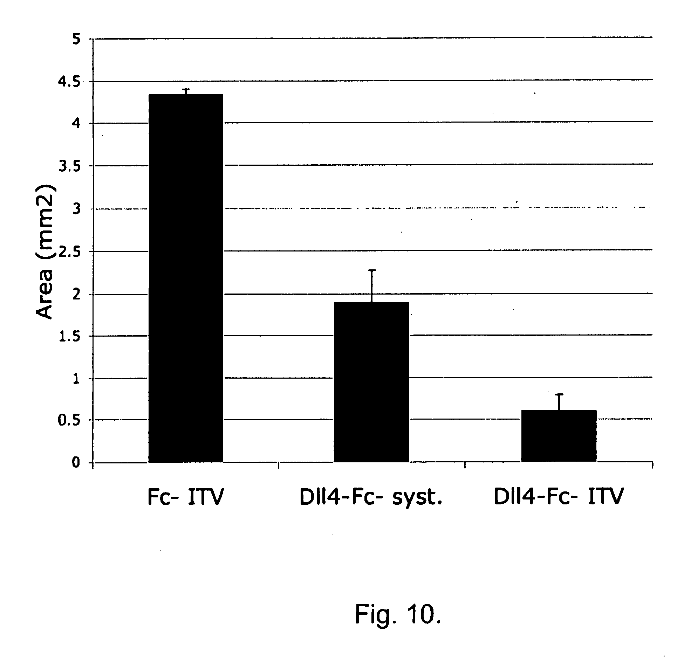

Fig. 10. Systemic delivery of DII4-Fc reduces or prevents retinal blood vessel loss. 4.1 mcg of hDII4-hFc or 5 mcg of human hFc control protein was injected intravitreally (ITV) or hDII4-hFc was injected intraperitoneally at a dose of 25 mg per kg of the body weight at P7. Pups were placed into a 75% oxygen environment at P8. Retinal vasculature was analyzed at P9. Results were significantly different at the p<0.0001 level.

### DETAILED DESCRIPTION

Before the present methods are described, it is to be understood that this invention is not limited to particular methods, and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

### Definitions

By the phrase "DII4 antagonist" is meant an agent capable of inhibiting DII4 biological activity by blocking the interaction of DII4 with its receptors. Inhibition of DII4 activity may be through inhibition of receptor activation either with an antibody to DII4 or with a DII4 analog or fragment which binds but does not activate the receptor (nonproductive binding). Common inhibitors include but are not limited to antibodies, soluble receptors, oligonucelotide aptamers, peptides or other small molecule antagonists and their derivatives, and modified DII4 ligands which bind their Notch receptor but are unable to activate signaling through such binding. Other approaches include interfering with the expression of the gene encoding DII4 or blocking Notch receptor activation, for example with siRNAs or gamma secretase inhibitors, respectively.

A "neutralizing" or "blocking" antibody, is intended to refer to an antibody whose binding to DII4 results in inhibition of the biological activity of DII4. This inhibition of the biological activity of DII4 can be assessed by measuring one or more indicators of DII4 biological activity. These indicators of DII4 biological activity can be assessed by one or more of several standard *in vitro* or *in* vivo assays known in the art (see examples below). Preferably, the ability of an antibody to neutralize DII4 activity is assessed by inhibition of DII4 binding to a Notch receptor, such as Notch1 and Notch4.

### General Description

Notch signaling pathways are evolutionarily conserved, and play key roles in cell-fate determination and differentiation in many tissues during embryonic and postnatal development. Major components of the Notch pathway are expressed in the vasculature and genetic deletion of certain Notch pathway components, including Notch1, Notch1/Notch4, Jagged1, DII1, DII4, Hey1/Hey2 or presenilins, results in embryonic lethality associated with vascular remodeling defects. Although most of these genes are expressed in multiple tissue and cell types, DII4 is largely restricted to the vascular endothelium, suggesting that DII4 is a key ligand for Notch receptors in the vasculature.

During early embryonic development, genetic deletion of even a single DII4 allele produces severe vascular abnormalities that result in embryonic lethality in most mouse strains. Indeed, among the many genes involved in vasculogenesis and angiogenesis, haploid insufficiency has been reported to result in major vascular defects and embryonic lethality only for DII4 and VEGF-A. Early embryonic lethality precludes most experimental manipulations, making it difficult to precisely understand the role of DII4 during vascular development and in pathological settings.

To overcome this limitation, the effects of DII4 gene deletion were studied in mice of the ICR strain, in which haplo-insufficiency produces only limited embryonic lethality. The vascular phenotype observed in these mutant mice was compared to that obtained in wild-type mice in which DII4/Notch signaling was selectively inhibited by intravitreal injection of a soluble inhibitor of DII4, DII4-Fc, or a neutralizing polyclonal antibody against the extracellular domain of DII4. For these experiments, the retina was selected as a model system in which to study DII4 biology, because the retinal vasculature develops postnatally in a stereotypic manner that is highly organized, temporally and spatially.

The murine model of oxygen-induced ischemic retinopathy (OIR) is a well characterized model of pathological neovascularization associated with elevated expression of the essential pro-angiogenic factor, VEGF (Smith et al. 1994 Invest Ophthalmol Vis Sci 35:101-111; Neely et al. 1998 Am J. Pathol 153:665-670; Saint-Geniez et al. 2004 Int J Dev Biol 48:1045-1058) and thus relevant to pathological angiogenesis associated with diverse disease conditions (Ferrarra et al. 2005 Nature 438:967-974). Finally, the retinal vasculature is readily accessible to experimental manipulations, including intravitreal microinjections of experimental agents.

The experiments described below show that that during normal retinal vascular development, and in the OIR model, pharmacological suppression of DII4/Notch signaling markedly enhances angiogenic sprouting and promotes the formation of a denser primary capillary network. Consistent with this, it was found that endogenous DII4 expression is particularly prominent in the most active regions of vascular growth during both normal development and in the OIR model. Moreover DII4 inhibition markedly suppressed formation of the abnormal vasculature and prevented blood vessel occlusion and regression.

### DII4 Antagonists

DII4 antagonists include antibodies to DII4 and fragments thereof capable of blocking the binding of DII4 to a Notch receptor, for example Notch1; and proteins or protein fragments comprising the extracellular domain of DII4 which may be fused to a multimerizing component; peptides and peptibodies (see for example, US patent publication 2003/0229023 Oliner et al.).

DII4 antibodies. The phrase "immunoglobulin or antibody" as used herein refers to a mammalian, including human, polypeptide or protein comprising a framework region from an immunoglobulin gene or fragments thereof that specifically binds and recognizes an antigen, which, in the case of the present invention, is a DII4 protein or portion thereof. If the intended antibody or antibody-like protein will be used as a mammalian therapeutic, immunoglobulin binding regions should be derived from the corresponding mammalian immunoglobulins. If the molecule is intended for non-therapeutic use, such as for diagnostics and ELISAs, the immunoglobulin binding regions may be derived from either human or non-human mammals, such as mice. The human immunoglobulin genes or gene fragments include the kappa, lambda, alpha, gamma, delta, epsilon, and mu constant regions, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. Within each IgG class, there are different isotypes (eg. IgG₁, IgG₂, etc.) as well as allotypes thereof.

An exemplary immunoglobulin (antibody) structural unit of human IgG, comprises a tetramer. Each tetramer is composed of two identical pairs of polypeptide chains, each pair having one light chain (about 25 kD) and one heavy chain (about 50-70 kD). The N-terminus of each chain defines a variable region of about 100-110 or more amino acids primarily responsible for antigen recognition. The terms "variable light chain" (V_{L}) and variable heavy chain (V_{H}) refer to these light and heavy chains respectively.

Antibodies exist as intact immunoglobulins, or as a number of well-characterized fragments produced by digestion with various peptidases. For example, pepsin digests an antibody below the disulfide linkages in the hinge region to produce F(ab)'₂, a dimer of Fab which itself is a light chain joined to V_{H}-C_{H} by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region. While various antibody fragments are described in terms of the digestion of an intact antibody, one of skill will appreciate that such fragments may be synthesized de novo either chemically or by using recombinant DNA methodology. Thus, the terms antibody, as used herein, also includes antibody fragments either produced by the modification of whole antibodies, or those synthesized *de novo* using recombinant DNA methodologies, or generated via display libraries such as phage, *E*. *coli* or yeast display libraries (see, for example, McCafferty et al. (1990) Nature 348:552-554).

DII4 analogs or protein fragments. The DII4 antagonist may be a DII4 fragment optionally connected to a multimerizing component. In specific embodiments, a DII4 fragment may be fused to a multimerizing component such as an immunoglobulin domain, a truncated immunoglobulin domain, or an amino acid sequence between 1 to about 500 amino acids in length, optionally comprising at least one cysteine residue. In a preferred embodiment, the multimerizing component is an immunoglobulin domain, preferably an Fc domain, e.g., a human Fc (SEQ ID NO:5). The protein or protein fragment may optionally comprise a signal sequence, which may comprise any sequence known to a skilled artisan for directing secretion of a polypeptide or protein from a cell, including natural or synthetic sequences. Generally, a signal sequence is placed at the beginning or amino-terminus of the fusion protein . Such a signal sequence may be native to the cell, recombinant, or synthetic.

The extracellular domain of Dll4 is composed of a Delta/Serrate/Lag-2 (DSL) domain and eight epidermal growth factor (EGF)-like tandem repeats. Generally, the EGF domains are recognized as occurring at about position 218-251 (domain 1), 252-282 (domaine 2). 284-322 (domain 3), 324-360 (domain 4), and 362-400 (domain 5), with the DSL domain at about position 173-217 and the N-terminal domain at about position 27-172 of hDll4 (SEQ ID NO:2). In specific embodiments, the Dll4 antagonist comprises about amino acid 27-172, 27-217, 218-400, 218-360, 218-322, or 218-282 of SEQ ID NO:2, optionally fused to hFc (SEQ ID NO:5).

### Methods of Administration

The present disclosure relates to methods of treatment comprising administering to a subject an effective amount of an agent described herein. In a preferred aspect, the agent is substantially purified (e.g., substantially free from substances that limit its effect or produce undesired side-effects), The subject is preferably an animal, e.g., such as a cow, pig, horse, chicken, cat, dog, etc., and is preferably a mammal, and most preferably human. In a specific embodiment, it may be desirable to administer the pharmaceutical compositions described herein locally to the area in need of treatment; this may be achieved, for example, and not by way of limitation, by local infusion during surgery, topical application, e.g., by injection, by means of a catheter, or by means of an implant.

The methods described herein may be advantageously performed by administration to the area in need of treatment by local administration, including intravitreal, intraocular, perlocular, subconjunctival, juxtascieral, subtenon, or topical administration.

### Combination Therapies

In various embodiments, the method described herein is accomplished with a Dll4 antagonist, such as a Dll4 antibody, in combination with one or more additional compounds or therapies or medical procedures. For example, suitable therapeutic agents for use In combination, either alternating or simultaneously, include fusion proteins capable of binding and inhibiting the activity of vascular endothelial growth factor (VEGF) (see U.S. 7, 070,959 and 7,087,411), immunosuppressive agents such as corticosteroids, dexamethasone, cyclosporin A, FK506, or antimetabolic agents, (see Barker, NH, et al., (2000) Clin Exp Opthal 28:357-360). Other suitable therapeutic agents for use in combination, either alternating or simultaneously, with DII4 antagonists may include agents that can block the biological activity of other VEGF family members such as VEGF-C and VEGF-D.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (*e.g*., amounts, areal measurements, etc.) but some experimental errors and deviations should be accounted for.

### Example 1. Effect of genetic deletion of a single DII4 allele on blood vessel sprouting in the developing retinal vasculature.

An investigation was undertaken to determine the effects of Dll4 partial genetic deficiency on blood vessel sprouting during normal developmental retinal angiogenesis.

Animals. Velocigene^{™} technology (Valenzuela et al. (2003) Nat. Biotechnol. 21:652-9; US Patent 6,586,251) was used to replace the entire Dll4 coding region with the β-gatactosidase reporter gene in C57BL/6:129 hybrid mouse embryonic stem cells. Chimeric males were bred to ICR females. Dll4^{+lacZ} mice backcrossed for 3 generations to ICR (87.5% ICR) were used for this study.

Histochemistry and Immunostaining. Mouse pups were humanely euthanized at P7. Eyes were enucleated, and retinas were dissected, fixed overnight with 4% paraformaldehyde, stained with FITC-labeled Griffonia simplicifolia (GS) lectin I (Vector Laboratories, Burlingame, CA), and flat-mounted. Images were taken by using a Nikon (Melville, NY) microscope.

Quantifications. Measurements were performed using the Scion Image software. Each experimental condition was assayed at least in triplicate. Student's t-test and two-way analysis of variance were used to assess statistical significance.

Results. Peripheral plexus was much denser in Dll4^{+lacZ} mice than in wild-type littermates. Higher-power views highlighted that the denser peripheral plexus in the retinas of Dll4^{+lacZ} mice consisted of capillaries that were larger in diameter, more highly interconnected and hyperfused, so that in some areas the vessels coalesced to form a syncytium, and, in addition, that there were many more sprouts at the growing front (Fig. 1); filopodia also were observed in more interior portions of the plexus at a higher than normal frequency. Quantitative analyses revealed that, compared to wild type controls, retinas of Dll4^{+lacZ} mice showed a 68% increase in the number of sprouts at the growing front of the superficial retinal vasculature (Fig. 1), as well more than a two fold increase in the number of capillary interconnections per unit area, resulting in a significant increase in the vascular coverage. Despite the marked morphologic changes, intravascular injection of fluoresceinated lectin completely filled the developing superficial vascular plexus, except for the filopodia extending from the tip cells, in Dll4^{+lacZ} mice as in wild-type mice, indicating that all components of the developing vasculature had lumens and were functional.

### Example 2. Effect of Dll4/Notch inhibition with Dll4-Fc or anti-Dll4 antibody on the developing retinal vasculature.

An investigation was undertaken to determine the effects of Dll/Notch signaling pharmacological inhibition on endothelial cell proliferation and blood vessel sprouting during normal developmental retinal angiogenesis.

Antibodies and reagents. Dll4-Fc comprises the extracellular domain of mouse or human Dll4 and the Fc part of human IgG. Dll4-Fc was expressed in CHO cells and affinity purified by protein-A chromatography. Anti-Dll4 antibody was produced by immunization of rabbits with recombinant mDll4-hFc. The anti-serum was partially purified by protein-A chromatography prior to use.

Animals. C57/BI6 mice (Taconic) were used to study the effect of Dll4-Fc or neutralizing Dll4 antibody on developing retinal vasculature.

Intravitreal microinjections. Intravitreal microinjections (30 -100 nl) of the research compounds were made between the equator and the corneal limbus by using a Drummond Scientific (BroPA) nanoinjector equipped with a glass needle.

BrdU labeling. Proliferating cells were labeled by administration of BrdU (1 mg/kg i.p.) 20 h after intravitreal injection of hFc or Dll4-Fc. Retinas were harvested 4 h later and stained with ant-BrdU (Dako North America, Inc., Carpinteria, CA) and VE- Cadherin (BD PharMingen, San Diego, CA) antibodies.

Results. To study the effect of local intraretinal deficiency in Dll4/Notch signaling, and not secondary to an undetected systemic abnormality, a soluble version of Dll4 (termed Dll4-Fc) that binds Notch receptors without activating them, thereby blocking the actions of endogenous Dll4, or a neutralizing polyclonal antibody specific for the extracellular domain of Dll4, was injected into the vitreous of wild-type mice. Three days after intraocular administration of either Dll4 blocker, the retinal vessels exhibited morphologic changes that closely resembled the vascular abnormalities found in Dll4^{+/lacZ} mice, including dramatically increased blood vessel density and vessel caliber. Moreover, these characteristic morphologic changes occurred rapidly, being clearly evident within 24 hours.

BrdU labeling also showed an increase in endothelial cell proliferation within 24 h of Dll4 blockade (Fig. 2). The observed -17 % increase in the proliferation rate could yield more than 50% increase in cell number within three to four doubling times.

### Example 3. Effect of Dll4-Fc and anti-Dll4 antibody on retinal vascularization, perfusion and vascular abnormalities in mice with OIR.

An investigation was undertaken to determine the effects of the Dll4-Fc and anti-Dll4 antibody on the growth of retinal vessel, formation of vascular abnormalities and retinal perfusion in oxygen-induced ischemic retinopathy (OIR).

To determine whether Dll4/Notch signaling plays a role in pathologic angiogenesis as well as during normal development, the OIR model was utilized. In the OIR model, exposure of mouse pups to hyperoxia at P7 results in a rapid obliteration of capillaries in the central retina. Following return to room air at P12, the avascular zone becomes severely hypoxic, which in turn elicits extensive abnormal neovascularization, characterized by the ectopic growth of vessels into the vitreous (epiretinal vascular 'tufts') and the formation of abnormal arteriovenous shunts; central parts of the retina remain largely avascular for an extended period.

Animals and OIR model. C57/BI6 mice (Taconic) were used to study the effect of Dll4-Fc or neutralizing DII4 antibody on retinal neovascularization in OIR. OIR was produced following the method developed by Smith et al. (Invest. Ophthalmol. Vis. Sci. 1994, 35:101-111). Briefly, mouse pups and their dams were placed into a 75% oxygen environment from postnatal days (P) 7 to P12, and then returned to room air. Exposure to hyperoxia induces rapid vasoobliteration in the central retina. When mice are returned to room air (P12), the loss of vessels from the central retina results in severe hypoxia / ischemia which in turn stimulates the pathological vascular changes described above.

To label patent blood vessels, 50 ml of Texas red-labeled Lycopersicon esculentum (LE) lectin (1 mg/ml; Vector Laboratories, CA) was injected into the left cardiac ventricle and allowed to circulate for 5 min.

Results. Dll4/Notch inhibition with either Dll4-Fc or anti-Dll4 antibody ameliorated pathological neovascularization (Fig. 3A-B), stimulated growth of new blood vessel (Fig. 4A-B) and improved retinal re-perfusion (Fig. 5).

Dll4-Fc or anti-Dll4 antibody or a control protein (hFc) was injected intravitreally at P13, one day after the animals were returned to room air, well after vaso-obliteration was complete. When the retinas were evaluated at P17, administration of Dll4-Fc or anti-Dll4 antibody dramatically suppressed the ectopic growth of pathological neovascular tufts into the vitreous (Fig. 1), and also prevented the formation of abnormal arteriovenous shunts. The areas occupied by neovascular tufts was reduced by 43% in the retinas treated with Dll4-Fc and by 85% in the retinas treated with anti-Dll4 antibody both comparing to hFc treated control retinas.

Moreover, Dll4-Fc and anti-Dll4 antibody were found to stimulate more extensive sprouting of new vessels from capillaries and veins bordering the avascular zone, resulting in a more rapid re-growth of blood vessels into the central retina where the vasculature had been depleted thus reducing the avascular retinal area (Fig. 4A-B). The avascular areas were reduced by 43% in the retinas treated with Dll4-Fc and by 63% in the retinas treated with anti-Dll4 antibody. Particularly notable was the extensive capillary re-growth emanating from veins in the avascular zone of Dll4-Fc treated retinas. Thus, attenuation of Dll4/Notch signaling favored the extension of new vascular sprouts along the retinal surface, and obtunded the formation of epiretinal neovascularization, resulting in a more rapid reformation of the superficial vascular plexus. The new forming vessels were functional and exhibited improved retinal reperfusion as evident by 45% reduction of nonperfused areas in Dll4-Fc treated retinas (Fig. 5).

### Example 4. Effect of Dll4-Fc and anti-Dll4 antibody on retinal hypoxia/ischemia in the OIR model.

An investigation was undertaken to determine the effects of the Dll4-Fc and anti-Dll4 antibody on retinal hypoxia/ischemia in the OIR model of pathological neovascularization.

Excessive blood vessel growth under certain circumstances may interfere with normal blood circulation and reduce tissue oxygenation. To test whether Dll4/Notch inhibition can improve tissue oxygenation, HYPOXYPROBE™-1 (Chemicon) was used in a non-invasive assay to detect tissue hypoxia and determine the effect of Dll4-Fc treatment on retinal hypoxia/ischemia. HYPOXYPROBE™-1 was injected intraperitoneally at 100 mg/kg one hour prior to collecting the retinas for evaluation.

Results. The growth of functional new vessels following intravitreal administration of Dll4/Notch inhibitors effectively reduced tissue hypoxia/ischemia as evident by 69% and 30% reduction of hypoxyprobe-positive areas in Dll4-Fc or anti-Dll4 antibody treated retinas, respectively (Fig. 6A-B).

### Example 5. Genetic deletion of a single Dll4 allele reduces hyperoxia-induced vaso-obliteration.

An investigation was undertaken to determine the effects of Dll4 partial genetic deficiency on hyperoxia-induced blood vessel regression.

Animals. Dll4^{+/lacZ} mice backcrossed for 3 generations to ICR (87.5% ICR) were generated as described above. Due to a recessive (rd/rd) mutation the retinal photoreceptor cell layer starts to degenerate at P12 in ICR mice. Therefore, to obviate potential secondary effects of photoreceptor loss on the retinal vasculature, for evaluating later stages of retinal development and for all OIR experiments, Dll4^{+/lacZ} (87.5% ICR) mice were backcrossed to C57BL/6 to produce Rd/rd mice, which do not exhibit photoreceptor degeneration.

Mouse pups were placed into a 75% oxygen environment from postnatal days (P) 7 to P12. Retinas were harvested and retinal vasculature was analyzed in flat-mounts.

Results. Reduced expression of Dll4 in the retinal vasculature of Dll4^{+/lacZ} mice partially prevents hyperoxia-induced retinal blood vessel loss (Fig. 7). In the OIR model, exposure of mouse pups to hyperoxia at P7 results in a rapid occlusion and obliteration of capillaries in the central retina. To determine whether Dll4/Notch inhibition may have a protective effect on blood vessels, the effect of Dll4 partial genetic deficiency in Dll4^{+/lacZ} mice on hyperoxia-induced vasoobliteration was analyzed. Evaluating animals at P12 (so as to assess the extent of hyperoxic vaso-obliteration) it was found that vaso-obliteration was reduced by 40% in Dll4^{+/lacZ} mice comparing to wild-type littermate control animals, suggesting that Dll4 inhibitors may protect existing blood vessels from regression.

### Example 6. Effects of Dll4-Fc and anti-Dll4 antibody on hyperoxia-induced vaso-obliteration.

An investigation was undertaken to determine the effects of Dll4/Notch inhibition with Dll4-Fc and anti-Dll4 antibody on hyperoxia-induced blood vessel regression.

Animals. C57/BI6 mice (Taconic) were used to study the effect of Dll4-Fc or neutralizing Dll4 antibody on oxygen-induced retinal vaso-obliteration. Intravitreal microinjections of the research compounds were performed at postnatal day 8. At postnatal day 9 pups were placed into a 75% oxygen environment. Retinas were harvested 24 hours later and retinal vasculature was analyzed in flat-mounts.

Results. Intravitreal injection of Dll4-Fc or anti-Dll4 antibody dramatically reduced areas of obliterating vasculature by 97% and 41 % respectively (Fig. 8A-B).

### Example 7. Effects of Dll4-Fc and anti-Dll4 antibody on hyperoxia-induced blood vessel occlusion.

An investigation was undertaken to determine the effects of Dll4/Notch inhibition with Dll4-Fc and anti-Dll4 antibody on hyperoxia-induced blood vessel occlusion. All procedures were performed as described above.

Dll4-Fc and anti-Dll4 antibody treatment reduced non-perfused retinal areas by 40% and 29% respectively (Fig. 9A-B).

### Example 8. Effects of systemic administration of Dll4-Fc on hyperoxia-induced blood vessel regression.

An investigation was undertaken to determine the effects of systemic treatment with Dll4-Fc on hyperoxia-induced blood vessel regression.

4.1 mcg of hDll4-hFc or 5 mcg of human hFc control protein was injected intravitreally (ITV) or hDll4-hFc was injected intraperitoneally at a dose of 25 mg per kg of the body weight at P7. Pups were placed into a 75% oxygen environment at P8 and retinal vasculature was analyzed at P9. All other procedures were performed as described above.

Results. Both local (intravitreal) and systemic (intraperitoneal) administration of Dll4-Fc reduced areas of obliterating vasculature by 86% and 56% respectively, indicating that independently of the route of administration Dll4 inhibitors can be effectively used to protect blood vessels from regression (Fig. 10).

## Claims

1. Use of a delta-like ligand 4 (Dll4) antagonist in the manufacture of a medicament for preventing or reducing blood vessel loss or promoting productive angiogenesis, or both, in a subject having an ischemic injury or vascular insufficiency wherein the Dll4 antagonist comprises an antibody or antibody fragment that specifically binds Dll4 and blocks the binding of Dll4 to a Notch receptor or comprises the extracellular domain of Dll4, optionally connected to a multimerizing component.

2. Use according to claim 1, wherein the Dll4 antibody or antibody fragment is polyclonal or monoclonal.

3. Use according to claim 2, wherein the antibody or antibody fragment is humanized, chimeric, or is a fully human antibody or antibody fragment.

4. Use according to claim 3, wherein the antibody or antibody fragment is a single chain antibody, an Fab, or an F(ab')₂.

5. Use according to claim 1, wherein the multimerizing component is an Fc domain.

6. Use according to any one of the preceding claims, wherein the ischemic injury or vascular insufficiency is selected from retinopathy of prematurity, ischemic retinopathy, retinal vein or artery occlusion, and diabetic retinopathy.

7. Use according to any one of claims 1 to 5, wherein the ischemic injury or vascular insufficiency is selected from cerebral ischemia, cardiac ischemia, ischemic conditions affecting the limbs and other organs or tissues, arteriovenous malformations, wound healing, placental insufficiency, arterial narrowing and occlusion, atherosclerosis, and systemic or pulmonary hypertension.

8. A delta-like ligand 4 (Dll4) antagonist for use in preventing or reducing blood vessel loss or promoting productive angiogenesis, or both, in a subject having an ischemic injury or vascular insufficiency, wherein the Dll4 antagonist comprises an antibody or antibody fragment that specifically binds Dll4 and blocks the binding of Dll4 to a Notch receptor or comprises the extracellular domain of Dll4, optionally connected to a multimerizing component.

9. The Dll4 antagonist for use according to claim 8 wherein said Dll4 antibody or antibody fragment is polyclonal or monoclonal.

10. The Dll4 antagonist for use according to claim 9, wherein the antibody or antibody fragment is humanized, chimeric, or is a fully human antibody or antibody fragment.

11. The Dll4 antagonist for use according to claim 10, wherein the antibody or antibody fragment is a single chain antibody, an Fab, or an F(ab')₂.

12. The Dll4 antagonist for use according to claim 8, wherein the multimerizing component is an Fc domain.

13. The Dll4 antagonist for use according to any one of claims 8 to 12, wherein the ischemic injury or vascular insufficiency is selected from retinopathy of prematurity, ischemic retinopathy, retinal vein or artery occlusion, and diabetic retinopathy.

14. The Dll4 antagonist for use according to any one of claims 8 to 11, wherein the ischemic injury or vascular insufficiency is selected from cerebral ischemia, cardiac ischemia, ischemic conditions affecting the limbs and other organs or tissues, arteriovenous malformations, wound healing, placental insufficiency, arterial narrowing and occlusion, atherosclerosis, and systemic or pulmonary hypertension.

15. Use according to any one of claims 1 to 7 or the Dll4 antagonist for use according to any of claims 8 to 14, wherein the subject treated is a human being.

## Patentansprüche

1. Verwendung eines Delta-like-Ligand-4(Dll4)-Antagonisten bei der Herstellung eines Medikaments zur Prävention oder Verringerung von Blutgefäßverlust oder zur Begünstigung von produktiver Angiogenese oder für beides, bei einem Subjekt, das eine ischämische Verletzung oder Gefäßinsuffizienz hat, wobei der Dll4-Antagonist einen Antikörper oder ein Antikörperfragment umfasst, der/das spezifisch Dll4 bindet und die Bindung von Dll4 an einen Notch-Rezeptor blockiert, oder die extrazelluläre Domäne von Dll4, umfasst, gegebenenfalls verbunden mit einer multimerisierenden Komponente.

2. Verwendung gemäß Anspruch 1, wobei der Dll4-Antikörper oder das Dll4-Antikörperfragment polyklonal oder monoklonal ist.

3. Verwendung gemäß Anspruch 2, wobei der Antikörper oder das Antikörperfragment humanisiert, chimär ist oder ein vollständig humaner Antikörper oder ein vollständig humanes Antikörperfragment ist.

4. Verwendung gemäß Anspruch 3, wobei der Antikörper oder das Antikörperfragment ein Einzelketten-Antikörper, ein Fab oder ein F(ab')₂ ist.

5. Verwendung gemäß Anspruch 1, wobei die multimerisierende Komponente eine Fc-Domäne ist.

6. Verwendung gemäß einem der vorangehenden Ansprüche, wobei die ischämische Verletzung oder die Gefäßinsuffizienz ausgewählt ist aus Retinopathie der Prämaturität, ischämischer Retinopathie, Retinavenen- oder - arterienokklusion und diabetischer Retinopathie.

7. Verwendung gemäß einem der Ansprüche 1 bis 5, wobei die ischämische Verletzung oder die Gefäßinsuffizienz ausgewählt ist aus zerebraler Ischämie, kardialer Ischämie, ischämischen Zuständen, die die Extremitäten und andere Organe oder Gewebe betreffen, arteriovenösen Fehlbildungen, Wundheilung, Placentainsuffizienz, Arterienverengung und -okklusion, Atherosklerose und systemischem oder pulmonalem Hochdruck.

8. Delta-like-Ligand-4(Dll4)-Antagonist zur Verwendung bei der Prävention oder Verringerung von Blutgefäßverlust oder bei der Begünstigung produktiver Angiogenese oder beidem bei einem Subjekt, das eine ischämische Verletzung oder eine Gefäßinsuffizienz hat, wobei der Dll4-Antagonist einen Antikörper oder ein Antikörperfragment umfasst, der/das spezifisch Dll4 bindet und die Bindung von Dll4 an einen Notch-Rezeptor blockiert, oder die extrazelluläre Domäne von Dll4, umfasst, gegebenenfalls verbunden mit einer multimerisierenden Komponente.

9. Dll4-Antagonist zur Verwendung gemäß Anspruch 8, wobei der Dll4-Antikörper oder das Dll4-Antikörperfragment polyklonal oder monoklonal ist.

10. Dll4-Antagonist zur Verwendung gemäß Anspruch 9, wobei der Antikörper oder das Antikörperfragment humanisiert, chimär ist oder ein vollständig humaner Antikörper oder ein vollständig humanes Antikörperfragment ist.

11. Dll4-Antagonist zur Verwendung gemäß Anspruch 10, wobei der Antikörper oder das Antikörperfragment ein Einzelketten-Antikörper, ein Fab oder ein F(ab')₂ ist.

12. Dll4-Antagonist zur Verwendung gemäß Anspruch 8, wobei die multimerisierende Komponente eine Fc-Domäne ist.

13. Dll4-Antagonist zur Verwendung gemäß einem der Ansprüche 8 bis 12, wobei die ischämische Verletzung oder die Gefäßinsuffizienz ausgewählt ist aus Retinopathie der Prämaturität, ischämischer Retinopathie, Retinavenen- oder - arterienokklusion und diabetischer Retinopathie.

14. Dll4-Antagonist zur Verwendung gemäß einem der Ansprüche 8 bis 11, wobei die ischämische Verletzung oder die Gefäßinsuffizienz ausgewählt ist aus zerebraler Ischämie, kardialer Ischämie, ischämischen Zuständen, die die Extremitäten und andere Organe oder Gewebe betreffen, arteriovenösen Fehlbildungen, Wundheilung, Placentainsuffizienz, Arterienverengung und -okklusion, Atherosklerose und systemischem oder pulmonalem Hochdruck.

15. Verwendung gemäß einem der Ansprüche 1 bis 7 oder Dll4-Antagonist zur Verwendung gemäß einem der Ansprüche 8 bis 14, wobei das Subjekt, das behandelt wird, ein Mensch ist.

## Revendications

1. Utilisation d'un antagoniste du ligand Dll4 (ligand de type Delta 4 ou "Delta-like ligand 4") dans la fabrication d'un médicament destiné à prévenir ou réduire la perte de vaisseaux sanguins ou à promouvoir une angiogenèse productive, ou ayant ces deux visées, chez un sujet présentant une lésion ischémique ou une insuffisance vasculaire, pour laquelle l'antagoniste de Dll4 comprend un anticorps ou un fragment d'anticorps qui se lie spécifiquement au ligand Dll4 et empêche le ligand Dll4 de se lier au récepteur Notch, ou comprend le domaine extracellulaire de Dll4, en option raccordé à un composant multimérisable.

2. Utilisation conforme à la revendication 1, pour laquelle l'anticorps ou le fragment d'anticorps anti-Dll4 est polyclonal ou monoclonal.

3. Utilisation conforme à la revendication 2, pour laquelle l'anticorps ou le fragment d'anticorps est humanisé, est chimérique, ou est un anticorps ou fragment d'anticorps pleinement humain.

4. Utilisation conforme à la revendication 1, pour laquelle l'anticorps ou le fragment d'anticorps est un anticorps à chaîne simple ou un fragment Fab ou Fab'₂.

5. Utilisation conforme à la revendication 1, pour laquelle le composant multimérisable est un domaine Fc.

6. Utilisation conforme à l'une des revendications précédentes, pour laquelle la lésion ischémique ou l'insuffisance vasculaire est choisie parmi une rétinopathie des prématurés, une rétinopathie ischémique, une occlusion de veine ou d'artère rétinienne, et une rétinopathie diabétique.

7. Utilisation conforme à l'une des revendications 1 à 5, pour laquelle la lésion ischémique ou l'insuffisance vasculaire est choisie parmi une ischémie cérébrale, une ischémie cardiaque, les états d'ischémie affectant les membres et d'autres organes ou tissus, les malformations artério-veineuses, une cicatrisation, une insuffisance placentaire, le rétrécissement ou l'occlusion d'une artère, une athérosclérose, et une hypertension artérielle systémique ou pulmonaire.

8. Antagoniste du ligand Dll4 (ligand de type Delta 4 ou "Delta-like ligand 4") pour utilisation dans la prévention ou la réduction de la perte de vaisseaux sanguins ou la promotion d'une angiogenèse productive, ou dans ces deux visées, chez un sujet présentant une lésion ischémique ou une insuffisance vasculaire, lequel antagoniste de Dll4 comprend un anticorps ou un fragment d'anticorps qui se lie spécifiquement au ligand Dll4 et empêche le ligand Dll4 de se lier au récepteur Notch, ou comprend le domaine extracellulaire de Dll4, en option raccordé à un composant multimérisable.

9. Antagoniste du ligand Dll4 pour utilisation conforme à la revendication 8, dans lequel ledit anticorps ou fragment d'anticorps anti-Dll4 est polyclonal ou monoclonal.

10. Antagoniste du ligand Dll4 pour utilisation conforme à la revendication 9, dans lequel l'anticorps ou le fragment d'anticorps est humanisé, est chimérique, ou est un anticorps ou fragment d'anticorps pleinement humain.

11. Antagoniste du ligand Dll4 pour utilisation conforme à la revendication 10, dans lequel l'anticorps ou le fragment d'anticorps est un anticorps à chaîne simple ou un fragment Fab ou Fab'₂.

12. Antagoniste du ligand Dll4 pour utilisation conforme à la revendication 8, dans lequel le composant multimérisable est un domaine Fc.

13. Antagoniste du ligand Dll4 pour utilisation conforme à l'une des revendications 8 à 12, pour lequel la lésion ischémique ou l'insuffisance vasculaire est choisie parmi une rétinopathie des prématurés, une rétinopathie ischémique, une occlusion de veine ou d'artère rétinienne, et une rétinopathie diabétique.

14. Antagoniste du ligand Dll4 pour utilisation conforme à l'une des revendications 8 à 11, pour lequel la lésion ischémique ou l'insuffisance vasculaire est choisie parmi une ischémie cérébrale, une ischémie cardiaque, les états d'ischémie affectant les membres et d'autres organes ou tissus, les malformations artério-veineuses, une cicatrisation, une insuffisance placentaire, le rétrécissement ou l'occlusion d'une artère, une athérosclérose, et une hypertension artérielle systémique ou pulmonaire.

15. Utilisation conforme à l'une des revendications 1 à 7, ou antagoniste du ligand Dll4 pour utilisation conforme à l'une des revendications 8 à 14, pour laquelle ou lequel le sujet traité est un être humain.
